# EUROPEAN PATENT APPLICATION

(11) **EP 4 484 491 A1**
(43) Date of publication of application: **01.01.2025**
(21) Application number: 23912801.0
(22) Date of filing: 22.12.2023
(51) Int. Cl.: C08L 23/08, C08J 5/18

(54) **POLYETHYLENE RESIN COMPOSITION**

(30) Priority: 29.12.2022 KR 20220189078; 20.12.2023 KR 20230187173
(71) Applicant: LG Chem, Ltd., Yeongdeungpo-gu Seoul 07336 (KR)
(72) Inventor: YOON, Jiwon, Daejeon 34122 (KR); KIM, Na Un, Daejeon 34122 (KR); CHOI, Yi Young, Daejeon 34122 (KR)
(74) Representative: Hoffmann Eitle
(86) International application number: PCT/KR2023/021364
(87) International publication number: WO 2024/144126

(57) **Abstract**

Provided are a polyethylene resin composition having excellent stretchability and physical properties, and a biaxially stretched film using the same.

## Description

### [TECHNICAL FIELD]

### Cross-reference to Related Application

The present application is based on, and claims priority from, Korean Patent Application Nos. 10-2022-0189078 and 10-2023-0187173, filed on December 29, 2022 and December 20, 2023, respectively, the disclosures of which are hereby incorporated by reference herein in their entirety.

The present invention relates to a polyethylene resin composition having excellent stretchability and physical properties.

### [BACKGROUND ART]

With the recent increase in consumer concern for the environment, single-material packaging that is easy to recycle is attracting attention. Representatively, in the food packaging and distribution industry, there is a trend to manufacture "All-PE" films using only polyethylene (PE) which is a general-purpose material.

Previously, composite materials with BOPA, BOPET, BOPP, etc. applied to a printing layer have been used in multi-layer film packaging materials. In order to replace the composite materials by PE, they require higher physical properties than existing PE blown films.

Meanwhile, biaxially oriented polyethylene (BOPE) film is manufactured by stretching a cast sheet in both the machine direction (MD) and the transverse direction (TD), and has remarkably better tensile strength, impact strength, and transparency than existing blown films.

The key to developing high-density polyethylene (HDPE) for BOPE is that biaxial stretching from MD stretching to TD stretching must be performed.

Accordingly, research and development is needed to prepare a polyethylene or polyethylene resin composition with excellent stretchability.

### [DETAILED DESCRIPTION OF THE INVENTION]

### [TECHNICAL PROBLEM]

In order to solve the problems of the prior art, there is provided a polyethylene resin composition exhibiting excellent stretchability and physical properties through control of the crystal structure.

There is also provided a biaxially stretched film which exhibits excellent stretching ratio and strength properties, and is free from defects other than shrinkage when stretched in the TD direction by using the polyethylene resin composition.

### [TECHNICAL SOLUTION]

In order to achieve the above objects, there is provided a polyethylene resin composition which includes an ethylene/C4 to C10 alpha olefin copolymer and satisfies the following requirements of (a1) to (a3):
(a1) when a relative content of a peak area according to a melting temperature (Tm) (°C) is measured using SSA (Successive Self-nucleation and Annealing) analysis, a ratio (W_{LC}/W_{HC}) of a low-crystalline polymer content (W_{LC}) in a low temperature range of Tm of 100°C or lower to a high-crystalline polymer content (W_{HC}) in a high temperature range of Tm of 120°C or higher: 0.1 or more,
(a2) a density measured in accordance with the ASTM D1505 standard: 0.940 g/cm³ or more, and
(a3) a melt index (MI_{2.16}) measured at a temperature of 190°C under a load of 2.16 kg in accordance with the ASTM D1238 standard: 0.1 g/lOmin to 10 g/lOmin.

There is also provided a stretched film, specifically, biaxially stretched film, including the polyethylene resin composition.

### [ADVANTAGEOUS EFFECTS]

According to the present invention, provided is a polyethylene resin composition exhibiting excellent stretchability and physical properties.

Provided is also a biaxially stretched film which exhibits excellent stretching ratio and strength properties, and is free from defects other than shrinkage when stretched in the TD direction by using the polyethylene resin composition.

### [BRIEF DESCRIPTION OF THE DRAWINGS]

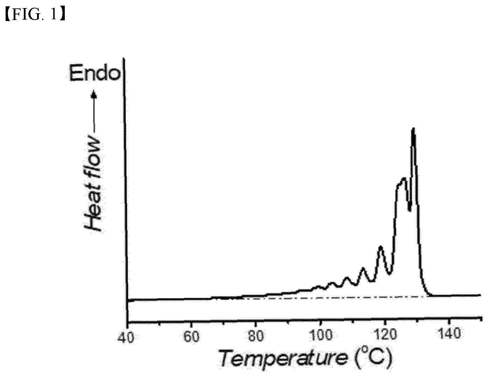

FIG. 1 is a graph showing the result of successive self-nucleation and annealing (SSA) analysis of a polyethylene resin composition according to Example 1.

### [DETAILED DESCRIPTION OF THE EMBODIMENTS]

As used herein, the terms "the first", "the second", and the like are used to describe a variety of components, and these terms are merely employed to differentiate a certain component from other components.

Further, the terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, numbers, steps, components, or combinations thereof, and do not exclude the existence or the possibility of addition of one or more different characteristics, numbers, steps, components or combinations thereof beforehand.

The present invention may be variously modified and have various forms, and specific exemplary embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

Hereinafter, a polyethylene resin composition of the present invention will be described in detail.

A polyethylene resin composition according to the present invention includes an ethylene/C4 to C10 alpha olefin copolymer and satisfies the following requirements of (a1) to (a3):
(a1) when a relative content of a peak area according to a melting temperature (Tm) (°C) is measured using SSA (Successive Self-nucleation and Annealing) analysis, a ratio (W_{LC}/W_{HC}) of a low-crystalline polymer content (W_{LC}) in a low temperature range of Tm of 100°C or lower to a high-crystalline polymer content (W_{HC}) in a high temperature range of Tm of 120°C or higher: 0.1 or more,
(a2) a density measured in accordance with the ASTM D1505 standard: 0.940 g/cm³ or more, and
(a3) a melt index (MI_{2.16}) measured at a temperature of 190°C under a load of 2.16 kg in accordance with the ASTM D1238 standard: 0.1 g/lOmin to 10 g/lOmin.

The polyethylene resin composition according to the present invention has a specific peak area distribution when a crystal distribution of the polymer, that is, a distribution of the peak area according to the melting temperature (Tm) is measured using SSA (Successive Self-nucleation and Annealing) analysis.

Usually, biaxially oriented polyethylene (BOPE) film is manufactured by stretching a cast sheet in both machine direction (MD) and transverse direction (TD). As described, since biaxial stretching must be performed from MD stretching to TD stretching, PE for BOPE requires excellent stretchability. In order to ensure stretchability in PE, a structure must exist that allows interlamellar lateral linking between lamellae within the PE so that secondary crystallization occurs as late as possible in MD stretching, followed by subsequent TD stretching.

The polyethylene resin composition according to the present invention is a semicrystalline polymer, and may include crystalline and amorphous regions. Specifically, in the crystalline region, a polymer chain including an ethylene repeating unit or an alpha olefin repeating unit is folded to form a bundle, thereby forming a crystalline domain (or a segment) in the form of lamella.

SSA is a method of preserving crystals crystallized at corresponding temperatures at each stage by a rapid cooling at end of each stage while lowering a temperature in a stepwise manner using a differential scanning calorimeter (DSC). In other words, the polyethylene resin composition is completely melted by heating, and then cooled to a specific temperature (T), and when slowly annealed, the lamellas that are unstable at the corresponding temperature (T) are still in a melted state, and only the stable lamellas crystallize. At this time, the stability at the corresponding temperature (T) depends on the thickness of the lamellae, and the thickness of the lamellae depends on the chain structure. Accordingly, when such a heat treatment is performed in a stepwise manner, the lamella thickness and its distribution according to a polymer chain structure may be quantitatively measured, and accordingly, the distribution of each melting peak area may be measured.

In the SSA analysis, the higher crystal content of the low-temperature region means the higher content of crystals capable of melting behavior at a low temperature. Therefore, secondary crystallization may occur relatively slowly, which is advantageous for TD stretching. On the other hand, this is also affected by the decrease in the relative crystal content of the high temperature region. However, when the crystal content in the high temperature region is lowered, physical properties such as stiffness, etc., which are critical to the film, are restricted. Accordingly, in the present invention, stretchability was secured by limiting the secondary crystallization rate, which is a prerequisite for enabling TD stretching, by controlling the crystal weight ratio.

Specifically, when a relative content of a peak area according to a melting temperature (Tm) (°C) is measured using SSA analysis, a ratio (W_{LC}/W_{HC}) of a low-crystalline polymer content (W_{LC}) in a low temperature range of Tm of 100°C or lower to a high-crystalline polymer content (W_{HC}) in a high temperature range of Tm of 120°C or higher is 0.1 or more, and thus the polyethylene resin composition according to the present invention may exhibit superior stretchability and physical properties, as compared to the existing polyethylene resin composition having the same density and melt index.

In the present invention, the SSA analysis may be performed by heating the polyolefin to a first heating temperature of 140°C to 180°C using a differential scanning calorimeter, holding for 15 minutes to 30 minutes, and then cooling at 28°C to 32°C, and repeating heating-holding-cooling until the final heating temperature reaches 50°C to 54°C while lowering the heating temperature stepwise wherein n+1^{th} heating temperature is set to 3°C to 7°C lower than the n^{th} heating temperature, and lastly, cooling from 50°C to 30°C, and then heating to 180°C, and measuring a thermogram by observing a change of heat quantity.

More specifically, the SSA may be performed by the following steps of i) to v):
i) heating the polyethylene to 180°C using a differential scanning calorimeter and then holding (isothermal) for 20 minutes to remove all thermal history before measurement;
ii) cooling from 180°C to 140°C, holding (isothermal) for 20 minutes, and then lowering the temperature to 30°C and holding for 1 minute;
iii) heating (annealing) to 135°C, which is 5°C lower than 140°C, and holding (isothermal) for 20 minutes, and then lowering (cooling) the temperature to 30°C;
iv) repeating heating-holding-cooling until the heating temperature reaches 50°C while gradually lowering the heating temperature, wherein n+1^{th} heating temperature (annealing temperature) is 5°C lower than n^{th} heating temperature, and the heating rate, holding time, and cooling temperature are the same; and
v) lastly, cooling from 50°C to 30°C, and then heating to 180°C at a rate of 10°C/min, and holding for 1 minute.

For example, the SSA analysis in the present invention may be performed according to the following method. First, the polyethylene resin composition is initially heated to 180°C at a rate of 20°C/min using a differential scanning calorimeter (device name: DSC8000, manufacturer: PerkinElmer), and then held for 20 minutes to remove all the thermal history of the sample before measurement. The temperature is lowered from 180°C to 140°C and held for 20 minutes, and then lowered to 30°C and held for 1 minute.

Next, the polyethylene resin composition is heated to a temperature (135°C), which is 5°C lower than the initial heating temperature of 140°C, held for 20 minutes, and cooled to 30°C. After holding for 1 minute, the temperature is increased again. In this manner, the n+1^{th} heating temperature is held at a temperature of 5°C lower than the n^{th} heating temperature, the holding time and cooling temperature are the same, and the heating-holding-cooling process is repeated while gradually lowering the heating temperature to 50°C. At this time, the heating and cooling rates are adjusted to 20°C/min, respectively.

Lastly, after cooling from 50°C to 30°C, heating-holding-cooling is repeated, and heating is performed at a heating rate of 10°C/min from 30°C to 180°C to quantitatively analyze the distribution of the formed crystals, followed by holding for 1 minute, and a thermogram is measured by observing a change of heat quantity.

When heating-holding-cooling is repeated for the polyethylene resin composition of the present invention by the SSA method, peaks appear at each temperature, and a relative content of the peak according to the melting temperature range may be calculated therefrom. At this time, the relative content of the peak may be defined as a ratio of the peak area at the corresponding melting temperature range (100°C or lower, higher than 100°C to lower than 120°C, 120°C or higher) to the area of the crystal melting peak in the entire temperature range. Further, the area of the peak may be calculated through integration for the corresponding peak.

When calculated in the same manner as above, the polyethylene resin composition according to the present invention exhibits a characteristic that a ratio (=ratio of W_{LC}/W_{HC}) of the low-crystalline polymer content (W_{LC}) in the low-temperature range of Tm of 100°C or lower relative to the high-crystalline polymer content (W_{HC}) in the high-temperature region of Tm of 120°C or higher is 0.1 or more. More specifically, the ratio of W_{LC}/W_{HC} is 0.1 or more, or 0.105 or more, or 0.106 or more, and 0.15 or less, or 0.14 or less, or 0.135 or less, or 0.132 or less. As the polyethylene resin composition has a higher W_{LC}/W_{HC} ratio than the existing polyethylene resin composition, the crystals are in a molten state during the stretching process, which may be more advantageous for stretching.

Further, when the relative content of the peak area according to the melting temperature (Tm) was measured for the polyethylene resin composition using the SSA analysis, the low-crystalline polymer content (W_{LC}) in the low temperature range of Tm of 100°C or lower, based on the entire polymer, i.e., a ratio (S1/(S1+S2+S3)) of the peak area (S1) of the melting temperature of 100°C or lower, relative to the entire peak area, is 0.08 to 0.1, more specifically, 0.08 or more, or 0.083 or more, or 0.090 or more, and 0.1 or less, or 0.095 or less, or 0.092 or less.

Further, when the relative content of the peak area according to the melting temperature (Tm) was measured for the polyethylene resin composition using the SSA analysis, the medium-crystalline polymer content (W_{MC}) of Tm of higher than 100°C and lower than 120°C, based on the entire polymer, i.e., a ratio (S2/(S1+S2+S3)) of the peak area (S2) of the melting temperature of 100°C or lower, relative to the entire peak area, is 0.01 to 0.25, more specifically, 0.01 or more, or 0.02 or more, or 0.04 or more, or 0.2 or more, and 0.25 or less, or 0.23 or less, or 0.22 or less.

Further, when the relative content of the peak area according to the melting temperature (Tm) was measured for the polyethylene resin composition using the SSA analysis, the high-crystalline polymer content (W_{HC}) in the high temperature range of Tm of 120°C or higher, based on the entire polymer, i.e., a ratio (S3/(S1+S2+S3)) of the peak area (S3) of the melting temperature of 120°C or higher, relative to the entire peak area, is 0.5 to 0.9, more specifically, 0.5 or more, or 0.6 or more, or 0.65 or more, or 0.68 or more, or 0.7 or more, and 0.9 or less, or 0.88 or less, or 0.87 or less, or 0.8 or less.

In addition to the above-described crystal structure characteristics, the polyethylene resin composition according to the present invention has a high density of 0.940 g/cm³ or more. Accordingly, it may exhibit excellent strength characteristics. When the density is less than 0.940 g/cm³, physical properties, particularly, MD stiffness, may deteriorate when manufacturing a stretched film. More specifically, the density of the polyethylene resin composition may be 0.940 g/cm³ or more, or 0.945 g/cm³ or more, or 0.947 g/cm³ or more, and 0.950 g/cm³ or less, or 0.949 g/cm³ or less. At this time, the density is a value measured in accordance with ASTM D1505.

Further, the polyethylene resin composition according to the present invention satisfies the characteristics as described above while having a melt index (MI_{2.16}) of 0.1 g/10min or more, and 10 g/10min or less, as measured at a temperature of 190°C under a load of 2.16 kg in accordance with the ASTM D1238 standard. When the melt index of the polyethylene resin composition is less than 0.1 g/10 min, sheet stretchability and film processability may deteriorate due to an excessively low melt index, and when it exceeds 10 g/10 min, processability may deteriorate and the physical properties of the stretched film may deteriorate. More specifically, the melt index (MI_{2.16}) may be 0.1 g/lOmin or more, or 0.5 g/lOmin or more, or 0.9 g/lOmin or more, or 0.95 g/lOmin or more, or 0.97 g/10min, and 10 g/lOmin or less, or 5 g/lOmin or less, or 3 g/lOmin or less, or 1.5 g/lOmin or less, or 1.3 g/lOmin or less, or 1.24 g/10min or less.

Further, the polyethylene resin composition may have MFRR (MI₅/MI_{2.16}) of 3 or more, and 10 or less, which is a ratio obtained by dividing a melt index (MI₅) measured at a temperature of 190°C under a load of 5 kg by a melt index (MI_{2.16}) measured at a temperature of 190°C under a load of 2.16 kg in accordance with the ASTM D1238 standard. More specifically, MFRR (MI₅/MI_{2.16}) may be 3 or more, or 3.5 or more, or 3.9 or more, or 4 or more, and 10 or less, or 8 or less, or 5 or less, or 4.6 or less, or 4.52 or less.

Further, when the polyethylene resin composition is subjected to a stress test under conditions of 90°C, 1% stress, and a stress application time of 25 minutes, the stress relaxation time required for a stress reduction rate to reach 0.1% was 60 seconds or less.

More specifically, the stress relaxation time of the polyethylene resin composition is 60 seconds or less, or 59 seconds or less, or 58 seconds or less. As the initial stress relaxation occurs rapidly, the polymer mobility during stretching is high, which is advantageous for crystal structure rearrangement and stretching. Accordingly, the lower limit of the stress relaxation time of the polyethylene resin composition is not particularly limited. However, in terms of maintaining mechanical properties of a biaxially stretched film when manufacturing the film, the stress relaxation time of the polyethylene resin composition may be 1 second or more, or 10 seconds or more, or 30 seconds or more, or 40 seconds or more.

The stress relaxation time of the polyethylene resin composition may be obtained by a method described in Mater. Hpriz., 8, 2553-2561(2021).

Specifically, in the present invention, the polyethylene resin composition is manufactured into a 0.5 mm-thick sheet using a hot press (190°C), then cut in accordance with the ASTM D 882 standard to manufacture a specimen. A dynamic mechanical analysis (DMA) device is used to perform a stress test at 90°C under a tensile mode by pulling the lower part of the PE sheet with 1% stress for a stress application time of 25 minutes, and the time taken for the stress reduction rate to reach 0.1% compared to the initial stress is measured, and expressed as a stress relaxation time. Detailed measurement methods and conditions are as described in Experimental Example below.

The polyethylene resin composition according to the present invention has the peak area distribution according to the melting temperature as described above, as well as the high density and the optimal range of melt index, thereby exhibiting excellent stretchability and physical properties, and as a result, when manufacturing a biaxially stretched film, there is no risk of defects such as shrinkage, etc. when stretched in the TD direction, and the manufactured biaxially stretched film may exhibit excellent stretching ratio and tensile strength characteristics.

Further, the polyethylene resin composition according to the present invention specifically includes an ethylene/C4 to C10 alpha-olefin copolymer.

Specific examples of the C4 to C10 alpha-olefin monomer may include 1-butene, 1-pentene, 4-methyl-1-pentene, 1-hexene, 1-heptene, 1-octene, 1-decene, 1-undecene, 1-dodecene, 1-tetradecene, 1-hexadecene, 1-eicocene, etc., and two or more thereof may be used.

The ethylene/C4 to C10 alpha-olefin copolymer may be specifically an ethylene/1-butene copolymer or an ethylene/1-butene/1-octene copolymer.

Meanwhile, the polyethylene resin composition according to the present invention as described above may be prepared by a slurry polymerization method using a plurality of reactors.

Specifically, the polyethylene resin composition may be prepared by a preparation method including:
a first step of preparing a first polyethylene by introducing hydrogen into a first reactor in the presence of a Ziegler-Natta catalyst and performing a first slurry polymerization reaction of ethylene and a C4 to C10 alpha-olefin first comonomer; and
a second step of transferring the first polyethylene to a second reactor which is connected to the first reactor, then introducing ethylene alone or ethylene and a C4 to C10 alpha olefin second comonomer, and performing a second slurry polymerization reaction,
wherein a weight ratio of the total amount of ethylene introduced into the first and second reactors and the total amount of comonomers including the first and second comonomers is 1:0.015 or more,
a polymerization pressure (P1) during the first slurry polymerization reaction is higher than a polymerization pressure (P2) during the second slurry polymerization reaction, and the polymerization pressure (P2) is 2 kgf/cm² to 4 kgf/cm² during the second slurry polymerization reaction.

Accordingly, in the second reactor, the first polyethylene transferred from the first reactor is further polymerized by ethylene, or ethylene and the C4 to C10 alpha olefin comonomer which are introduced into the second reactor, thereby producing an ethylene/alpha olefin copolymer, leading to preparing a polyethylene resin composition satisfying the above-described physical property requirements.

The method of preparing the polyethylene resin composition according to the present invention is performed by slurry polymerization using a plurality of reactors, and therefore, two or more reactors, for example, two or more continuous stirred tank reactors (CSTR) may be used.

Specifically, in the above preparation method, the first reactor in which the first slurry polymerization reaction for preparing the first polyethylene occurs, and the second reactor which is connected to the first reactor and in which the second slurry polymerization reaction occurs may be used.

Further, the polymerization process in the first and second reactors is performed by a slurry polymerization process.

In a solution polymerization, a catalyst is activated through high-temperature and highpressure reaction, and therefore, the prepared resin composition exhibits deteriorated stretchability due to a narrow molecular weight distribution (MWD) and a low content of high molecular weight tails. In contrast, in the slurry polymerization process of the present invention, the polymerization is performed at a relatively low temperature, and therefore, it is possible to secure a molecular structure that has a wide MWD and contains a large amount of high molecular weight tails required for a polyethylene resin composition for producing a transparent film. As a result, the composition may exhibit excellent biaxial stretchability.

Further, in the preparation method according to the present invention, the total amount of ethylene and the total amount of comonomer introduced into the first and second reactors satisfy a weight ratio of 1:0.015 or more, and more specifically, a weight ratio of 1:0.015 to 1:0.1, or 1:0.02 to 1:0.1, or 1:0.02 to 1:0.05, or 1:0.02 to 1:0.035, or 1:0.02 to 1:0.031. When the total amount of ethylene and the total amount of comonomer introduced into the first and second reactors satisfy the above weight ratio range, it is possible to more easily achieve the physical properties of the polyethylene resin composition described above.

In addition to the control of the input amount of the monomers described above, only ethylene may be introduced without the input of the comonomer during the second slurry polymerization reaction, or the input amount of the first comonomer introduced into the first slurry polymerization reaction may be larger than the input amount of the second comonomer introduced into the second slurry polymerization reaction. In this case, along with the bimodal molecular weight distribution, an NCD structure with a high content of SCBs derived from the first comonomer in the low molecular weight region may be achieved. Due to the high SCB content in the low molecular weight region, fluidity of tie molecules between crystals increases, and as a result, fibril formation is suppressed during the stretching process, which may be more advantageous for stretching in the TD direction.

Specifically, a weight ratio of the second comonomer introduced during the second slurry polymerization process to the first comonomer introduced during the first slurry polymerization reaction (=the weight of the second comonomer introduced during the second slurry polymerization reaction/ the weight of the first comonomer introduced during the first slurry polymerization process) may be 0 or more and less than 1. When the weight ratio of the first and second comonomers is 1 or more, a polyethylene resin composition that satisfies the physical properties requirements described above, particularly, the NCD index requirement, is not prepared. More specifically, the weight ratio of the first and second comonomers is 0 (only ethylene monomer is introduced without the comonomer during the second slurry polymerization reaction), or the weight ratio may be more than 0, or 0.1 or more, or 0.2 or more, or 0.25 or more, or 0.266 or more, and less than 1, or 0.5 or less, or 0.3 or less.

Meanwhile, a C4 to C10 alpha-olefin monomer may be used as the second comonomer, and specific examples thereof are as described above.

Further, the second comonomer may be the same as or different from the first comonomer. Specifically, the second comonomer is different from the first comonomer, and in this case, the combined physical properties of the polyethylene resin composition according to the present invention may be more easily achieved. More specifically, the first comonomer may be 1-butene, and the second comonomer may be 1-octene.

Further, in the preparation method according to the present invention, the polymerization pressure (P1) during the first slurry polymerization reaction is higher than the polymerization pressure (P2) during the second slurry polymerization reaction. The bimodal molecular weight distribution and the NCD structure in polyethylene resin composition may be more easily achieved by more increasing the pressure condition during the first slurry polymerization reaction, in addition to controlling the input amount of comonomer described above. Specifically, a difference (P1-P2) between the pressure (P1) during the first slurry polymerization reaction and the pressure (P2) during the second slurry polymerization reaction may be 2 kgf/cm² or more, or 3 kgf/cm² or more, or 3.5 kgf/cm² or more, or 3.6 kgf/cm² or more, and 6 kgf/cm² or less, or 5.5 kgf/cm² or less, or 5 kgf/cm² or less, or 4.9 kgf/cm² or less.

Further, in the preparation method according to the present invention, the polymerization temperature (T1) during the first slurry polymerization reaction is higher than the polymerization temperature (T2) during the second slurry polymerization reaction. The bimodal molecular weight distribution and the NCD structure in the polyethylene resin composition may be more easily achieved by more increasing the temperature during the first slurry polymerization reaction, in addition to controlling the input amount of comonomer described above and the polymerization pressure. Specifically, a difference (T1-T2) between the polymerization temperature (T1) during the first slurry polymerization reaction and the polymerization temperature (T2) during the second slurry polymerization reaction may be 3°C or higher, or 4°C or higher, or 5°C or higher, 10°C or lower, or 8°C or lower, or 6°C or lower.

Hereinafter, each step will be described in detail.

In the method of preparing the polyethylene resin composition according to the present invention, the first step is a step of preparing a first polyethylene by introducing hydrogen into a first reactor in the presence of a Ziegler-Natta catalyst and performing a first slurry polymerization reaction of ethylene and a C4 to C10 alpha-olefin first comonomer.

The Ziegler-Natta catalyst used to prepare the first polyethylene may specifically be a magnesium-supported titanium catalyst. Further, the magnesium-supported titanium catalyst may further include halogenated hydrocarbon.

Specifically, the magnesium-supported titanium catalyst includes a solid magnesium carrier. Examples of the magnesium carrier may include magnesium, magnesium oxide, magnesium chloride, or silica-magnesia, etc., and any one thereof or a mixture of two or more thereof may be used. Among them, considering catalytic activity and ease of realizing optimal physical properties of the polyethylene resin composition, the magnesium carrier may be magnesium or magnesium chloride.

Meanwhile, the magnesium-supported titanium catalyst includes a titanium-containing compound represented by the following Chemical formula (1) as a main catalyst:

[Chemical formula 1] Ti(OR¹)ₘX¹₄₋ₘ

in Chemical formula 1,
R¹ is the same or different and an alkyl group having 1 to 10 carbon atoms,
X¹ is a halogen atom, more specifically, Cl, Br, or I, and
m is an integer of 0 to 4.

In Chemical formula 1, R¹ may be specifically an alkyl group having 1 or more, or 2 or more, or 3 or more carbon atoms, and 10 or less, or 8 or less, or 5 or less carbon atoms.

Further, X¹ may be specifically Br or Cl, and more specifically Cl.

Specific examples of the titanium-containing compound may include titanium tetrachloride (TiCl₄) or chlorinated titanium oxide, and any one thereof or a mixture of both may be used.

Further, the catalyst including the titanium-containing compound may include Mg:Ti at a molar ratio of 1:1 to 10:1, or 2:1 to 7:1, or 4:1 to 5:1, based on the content of Mg in the magnesium carrier and the content of Ti in the titanium-containing compound. When included within the above molar ratio range, superior catalytic activity may be achieved.

Further, the shape of the catalyst is not particularly limited, but it may be, for example, in the form of spherical fine particles.

Further, the average particle size of the catalyst may be 5 µm to 100 µm, more specifically, 5 µm or more, or 8 µm or more, or 10 µm or more, and 100 µm or less, or 50 µm or less, or 20 µm or less, or 13 µm or less.

Meanwhile, the average particle size of the catalyst in the present invention may be measured by scanning electron microscopy (SEM) observation. Specifically, after obtaining an SEM image in which 100 or more catalyst particles are observed, a random straight line is plotted, and the average particle size of the catalyst particles may be calculated from the length of the corresponding straight line, the number of particles contained in the straight line, and the magnification.

Further, the magnesium-supported titanium catalyst may further include halogenated hydrocarbon.

Halogenated hydrocarbon changes the electrical properties of titanium by coordinating around titanium, which acts as an active point, and the large volume of halogenated hydrocarbon creates sufficient three-dimensional space between titanium atoms, thereby greatly improving catalytic activity during polyethylene polymerization.

The halogenated hydrocarbon may be specifically an alkane having 1 to 12 carbon atoms, a cycloalkane having 3 to 12 carbon atoms, an alkene having 2 to 12 carbon atoms, an alkyne having 2 to 12 carbon atoms, or an aromatic hydrocarbon having 6 to 30 carbon atoms, which is substituted with one or more halogen groups. The halogen group may be fluoro, chloro, or bromo.

Specific examples may include bromoform, tetrachloroethane, hexachloroethane, pentachloroethane, 1,1,2,2-tetrachloroethane, 1-bromo-1-chloroethane, 1,2-dibromoethane, 1,2-dichloroethane, bromoethane, hexachloropropane, 1,2,3-trichloropropane, 1,2-dichloropropane, 1-chloropropane, 2-chloropropane, chlorobutane, dichlorobutane, 1-chloro-2-methylpropane, n-butyl chloride, tert-butyl chloride, 1-chloro-3-methylbutane, 1-chloropentane, 1,5-dichloropentane, bromopentane, neopentyl chloride, 1-chloroheptane, cyclopropyl bromide, cyclobutyl chloride, cyclohexyl chloride, cyclohexyl bromide, vinylidene chloride, 1,2,3,3-tetrachloropropene, 1,2-dibromo-1-propene, 1,3-dichloropropene, hexachloro-1,3-butadiene, 2-bromo-2-butene, propargyl chloride, chlorobenzene, tetrachlorobenzene, trichlorobenzene, dichlorobenzene, 4-chlorobenzyl chloride, benzyl chloride, or 1,1-dichloro-2-phenylcyclopropane, etc., and any one thereof or a mixture of two or more thereof may be used.

The halogenated hydrocarbon may be included in an amount of 0.1 mol or more, or 0.12 mol or more, or 0.13 mol or more, and 500 mol or less, or 100 mol or less, or 50 mol or less, or 10 mol or less, or 1 mol or less, or 0.5 mol or less, based on 1 mol of the titanium-containing compound as the main catalyst.

The Ziegler-Natta catalyst may be prepared by a preparation method including a carrier treatment step of mixing and reacting a raw material of the magnesium carrier with alcohol; and a titanium-containing compound supporting step of reacting the product resulting from the above step with the titanium-containing compound represented by Chemical formula 1, and further including the step of bringing into contact with and reacting with halogenated hydrocarbon during the carrier treatment step and the titanium-containing compound supporting step, or after the titanium-containing compound supporting step. Accordingly, the method of preparing the polyethylene resin composition according to the present invention includes, before the first step, the step of preparing the Ziegler-Natta catalyst, specifically, the carrier treatment step and the titanium-containing compound supporting step, and may further include, during the carrier treatment step and the titanium-containing compound supporting step, or after the titanium-containing compound supporting step, the step of bringing into contact with and reacting with halogenated hydrocarbon may be further included.

Specifically, the carrier treatment step for preparing the Ziegler-Natta catalyst may be performed by mixing and reacting the raw material of the magnesium carrier with alcohol under in a non-polar solvent.

Specific examples of the alcohol may include methanol, ethanol, 1-propanol, isopropanol, n-butanol, isobutanol, 1-pentanol, isopentanol, n-hexanol, 1-octanol, 2-ethyl-1-hexanol, etc., and any one thereof or a mixture of two or more thereof may be used. Among them, ethanol or 2-ethyl-1-hexanol may be used.

The alcohol may be used in an amount of 1 mol or more, or 1.5 mol or more, or 2 mol or more, and 10 mol or less, or 8 mol or less, or 6 mol or less, based on 1 mol of the raw material of the magnesium carrier.

Meanwhile, the raw material of the magnesium carrier may include the magnesium carrier itself, such as magnesium, magnesium oxide, magnesium chloride, or silica-magnesia, etc.; or magnesium alcoholate such as magnesium ethylate, etc., and any one thereof or a mixture of two or more thereof may be used. For example, when magnesium alcoholate is used as the raw material of the magnesium carrier, it is converted into magnesium chloride by titanium tetrachloride (TiCl₄) which is introduced as the main catalyst, and is included as a carrier in the final prepared catalyst.

The method of mixing the raw material of the magnesium carrier with alcohol may be specifically performed by adding the alcohol to a slurry which is prepared by mixing the raw material of the magnesium carrier with a non-polar solvent such as hexane, or adding, to the slurry, a solution in which the alcohol is dissolved in a non-polar solvent, and then stirring the solution at a temperature of 20°C to 150°C until it becomes a transparent solution. As a result of the above mixing, a homogeneous solution of the magnesium carrier is obtained, and in the solution, an adduct is formed in which crystals of the magnesium carrier are surrounded by alcohol molecules.

Next, the step of supporting the titanium-containing compound for preparing the Ziegler-Natta catalyst is performed by reacting the reaction product of the magnesium carrier and alcohol, which is generated in the carrier treatment step, with the titanium-containing compound.

Specifically, to the reaction product of the magnesium carrier and alcohol, which is generated in the carrier treatment step, the titanium-containing compound is directly added or a solution in which the titanium compound is dissolved in a non-polar solvent is added, and reacted at -50°C to 120°C or -20°C to 80°C.

The type and content of the titanium-containing compound are as described above.

Further, when the titanium-containing compound is introduced, the reaction product of the magnesium carrier and alcohol, which is generated in the carrier treatment step, may be stirred at 10 rpm to 500 rpm, or 50 rpm to 400 rpm.

Meanwhile, when the Ziegler-Natta catalyst is prepared, halogenated hydrocarbon is introduced when alcohol is added to the magnesium carrier in the carrier treatment step; halogenated hydrocarbon is introduced when the titanium-containing compound is introduced in the titanium-containing compound supporting step; or after completing the step of supporting the titanium-containing compound, the step of adding and reacting halogenated hydrocarbon may be further performed.

The type and content of the halogenated hydrocarbon are as described above.

The Ziegler-Natta catalyst prepared through the above preparation method may be used as it is during polyethylene polymerization, or may be used as a slurry in a non-polar solvent.

Further, the Ziegler-Natta catalyst may be used together with an organometallic compound represented by the following Chemical formula 2 as a cocatalyst.

[Chemical formula 2] R²ₙMX²₍₃₋ₙ₎

in Chemical formula 2,
M is selected from the group consisting of elements of groups IB, IIA, IIIB and IVB in the periodic table,
R² is the same or different and is an alkyl group having 1 to 10 carbon atoms,
X² is halogen, and
n is an integer of 1 to 3.

In Chemical formula 2, M is specifically aluminum, R² is an alkyl group having 1 to 5 carbon atoms or 2 to 4 carbon atoms, and X² is Cl or Br.

Specific examples of the organometallic compound represented by Chemical formula 2 may include triethyl aluminum (TEAL), methyl aluminum dichloride, methyl aluminum dibromide, dimethyl aluminum chloride, dimethyl aluminum bromide, propyl aluminum dichloride, propyl aluminum dibromide, butyl aluminum dichloride, butyl aluminum dibromide, dibutyl aluminum chloride, dibutyl aluminum bromide, isobutyl aluminum dichloride, isobutyl aluminum dibromide, diisobutyl aluminum chloride, diisobutyl aluminum bromide, hexyl aluminum dichloride, hexyl aluminum dibromide, dihexyl aluminum chloride, dihexyl aluminum bromide, octyl aluminum dichloride, octyl aluminum dibromide, dioctyl aluminum chloride, and dioctyl aluminum bromide, etc., and any one thereof or a mixture of two or more thereof may be used.

Meanwhile, since the cocatalyst affects the polymerization activity of the magnesium-supported catalyst, the polymerization activity of the catalyst may be further increased by controlling the content of cocatalyst. For example, with regard to the organometallic compound represented by Chemical formula 2, aluminum may be used in an amount of 3 mol or more, or 10 mol or more, or 25 mol or more, and 200 mol or less, or 100 mol or less per 1 mol of titanium in the catalyst.

The cocatalyst may be added to the polyethylene polymerization reaction after mixing with the Ziegler-Natta catalyst, or may be separately added before or after the addition of the Ziegler-Natta catalyst. Accordingly, the method of preparing the polyethylene resin composition according to the present invention may further include the step of adding the cocatalyst when preparing the first polyethylene in the first step.

Meanwhile, the polymerization reaction in the first step is a copolymerization reaction of ethylene and alpha olefin, and the input amounts of the monomers may be determined considering the physical properties of the first polyethylene and the final polyethylene resin composition to be achieved. For example, in the present invention, the content of the first comonomer introduced into the first reactor may be 0.01% by weight to 5% by weight, based on the total weight of ethylene introduced into the first reactor. When introduced in the above content range, the combined physical properties of the polyethylene resin composition described above may be easily realized, and as a result, an increase in the stretching ratio in the TD direction may be realized due to an increase in the low molecular and low crystalline content ratio.

In addition, the polymerization reaction in the first step is performed under the condition of introducing hydrogen gas.

Since the input amount of hydrogen gas affects the physical properties of the prepared copolymer, the input amount is appropriately determined depending on the physical properties to be achieved. For example, in the present invention, the total input amount of hydrogen gas may be 0.001% by weight to 0.5% by weight, based on the total weight of monomers including ethylene and alpha olefin comonomer which are introduced into the first and second reactors. When hydrogen gas is introduced in the above range, the combined physical properties of the polyethylene resin composition described above may be easily achieved. More specifically, hydrogen gas may be introduced in an amount of 0.001% by weight or more, or 0.01% by weight or more, or 0.05% by weight or more, or 0.07% by weight or more, and 0.5% by weight or less, or 0.3% by weight or less, or 0.1% by weight or less.

Further, during the polymerization reaction in the first step, the temperature and pressure conditions are as previously defined.

The first polyethylene is prepared through polymerization reaction under the above preparation conditions.

Next, the second step is performed in the second reactor which is connected to the first reactor.

Specifically, the first polyethylene prepared in the first step is transferred to the second reactor which is connected to the first reactor, ethylene and the C4 to C10 alpha olefin second comonomer are introduced, and the second slurry polymerization reaction is performed.

In the second reactor, the additional polymerization reaction occurs between the first polyethylene transferred from the first reactor and the ethylene or the C4 to C10 alpha olefin comonomer introduced into the second reactor.

The second slurry polymerization reaction may be carried out in the presence of a catalyst.

In this case, the applicable catalyst is a Ziegler-Natta catalyst, as described in the step 1. Accordingly, the Ziegler-Natta catalysts used in the first and second slurry polymerization reactions may be the same as or different from each other. When the same Ziegler-Natta catalyst is used in the first and second slurry polymerization reactions, it may be easier to control the physical properties of the polyethylene resin composition to be prepared, and accordingly, to achieve the above physical property requirements.

Further, the second slurry polymerization reaction may be carried out under conditions with hydrogen addition or without hydrogen addition. For example, when hydrogen gas is added, hydrogen gas may be added in an amount of 0.001% by weight to 0.05% by weight, based on the total weight of the monomers including the ethylene and the alpha olefin introduced into the second reactor.

In addition, the temperature and pressure conditions during the polymerization reaction in the second step are as defined above.

Meanwhile, the preparation method according to the present invention may further include the step of melting and extruding the obtained polymerization product after completing the polymerization reaction in the second step.

A pellet-type polyethylene resin composition may be prepared by the above melting and extrusion process.

Since the polymerization product after the polymerization reaction in the second step is obtained in the powder form, the types of applicable antioxidants are limited. Further, since the content of the antioxidants has large variations according to powder, articles manufactured using the same have large variations in their physical properties. In contrast, when the melting and extrusion process is performed, the components including antioxidants are uniformly mixed, and the resulting articles may have uniform physical properties.

Meanwhile, as used herein, a pellet or a pellet-type is a small particle or piece formed by extrusion of raw material, and includes all the shape classified as a pellet in the art, including circle, flat, flake, polygon, rod shapes, etc. The size of pellet is appropriately determined according to the use and shape, and is not specifically limited, but the pellet in the present invention is defined as having an average diameter of 2 mm or more so as to be distinguished from powder having a small average diameter of about 1 mm. In this regard, the "diameter" is the longest distance among any straight distances of the outer circumference surface of the pellet, and it may be measured using imaging microscope, etc.

The melt extrusion process may be performed using a common extruder, and the specific method and conditions thereof are not particularly limited as long as the morphological conditions of the pellet are satisfied.

In addition, during the melt extrusion, one or more additives such as antioxidants, neutralizing agents, slip agents, anti-blocking agents, UV stabilizers, antistatic agents, etc. may be added. Accordingly, the prepared polyethylene resin composition may further include one or more additives such as antioxidants, neutralizing agents, slip agents, anti-blocking agents, UV stabilizers, and antistatic agents, etc. Meanwhile, in the present invention, the additives do not affect the physical properties of the polyethylene resin composition, and may improve the processability and quality of manufactured articles when manufacturing the articles using the resin composition.

For example, the antioxidants may include phenolic antioxidants; phosphorus-based antioxidants; amine-based antioxidants, etc., and any one thereof or a mixture of two or more thereof may be used. Specific examples of the antioxidant may include phenolic antioxidants such as pentaerythritol tetrakis[3-(3,5-di-tert-butyl-4-hydroxyphenyl)propionate], or 1,3,5-trimethyl-2,4,6-tris(3,5-di-tert-butyl-4-hydroxybenzyl)benzene, etc.; phosphorus-based antioxidants such as tris(2,4-di-tert-butylphenyl)phosphite, bis(2,4-di-tert-butylphenyl)pentaerythritol diphosphite, and bis(2,4-dicumylphenyl)pentaerythritol diphosphite, etc.; or amine-based antioxidants such as phenylnaphthylamine, 4,4'-(α,α-dimethylbenzyl)diphenylamine, and N,N'-di-2-naphthyl-p-phenylenediamine, etc., and any one thereof or a mixture of two or more thereof may be used. In addition, commercially available Irganox^{™} 1010 (manufactured by BASF), Irganox^{™} 3114 (manufactured by BASF), Irganox^{™} 1076 (manufactured by BASF), Irgafos^{™} 168 (manufactured by BASF), Irgafos^{™} 626 (manufactured by BASF) or Cyanox^{™} 1790 (manufactured by CYTEC) may also be used. Among them, the polyethylene resin composition may include a mixture of a primary phenolic antioxidant and a secondary phosphorus-based antioxidant. In this regard, the primary phenolic antioxidant may be added in an amount of 50 ppm or more, or 200 ppm or more, or 250 ppm or more, and 500 ppm or less, or 300 ppm or less, based on the total weight of the polymerization product resulting from secondary polymerization. The secondary phosphorus-based antioxidant is added in an amount of 100 ppm or more, or 300 ppm or more, or 500 ppm or more, and 1000 ppm or less, or 700 ppm or less, based on the total weight of the polymerization product resulting from secondary polymerization. Therefore, the polyethylene resin composition may include the primary phenolic antioxidant in an amount of 50 ppm or more, or 200 ppm or more, or 250 ppm or more, and 500 ppm or less, or 300 ppm or less, and the secondary phosphorus-based antioxidant in an amount of 100 ppm or more, or 300 ppm or more, or 500 ppm or more, and 1000 ppm or less, or 700 ppm or less, based on the total weight of the resin composition.

Further, the neutralizing agent may include calcium stearate (Ca-St), calcium palmitate, zinc stearate, zinc palmitate, or hydrotalcite (magnesium aluminum hydroxy carbonate), etc., and any one thereof or a mixture of two or more thereof may be used. Further, commercially available DHT-4A (manufactured by KYOWA), etc. may be used. The neutralizing agent may be added in an amount of 100 ppm or more, or 300 ppm or more, or 500 ppm or more, and 1000 ppm or less, or 700 ppm or less, based on the total weight of the polymerization product resulting from secondary polymerization. Therefore, the polyethylene resin composition may include the neutralizing agent in an amount of 100 ppm or more, or 300 ppm or more, or 500 ppm or more, and 1000 ppm or less, or 700 ppm or less, based on the total weight of the resin composition.

Further, the melting and extrusion process may be performed according to common methods, for example, at an extrusion temperature of 180°C to 220°C, or 180°C to 210°C using an extruder such as a twin screw extruder.

The polyethylene resin composition satisfying the above-mentioned physical property requirements is prepared using the above-described preparation method. The prepared polyethylene resin composition exhibits excellent stretchability and physical properties, and there is no concern about defects such as breakage or shrinkage, etc. during stretching in the TD direction when manufacturing the biaxial stretched film. Further, the manufactured biaxial stretched film may exhibit excellent stretch ratio characteristics and tensile strength.

According to the present invention, provided is a stretched film, specifically, biaxially stretched film, manufactured using the above-described polyethylene resin composition.

The stretched film exhibits excellent stretchability and strength properties by including the polyethylene resin composition that satisfies the above-mentioned combined physical property requirements.

Specifically, the stretched film has an elongation of 1000% to 1500% and a stiffness of 850 MPa to 1000 MPa, as measured in accordance with ASTM D 882. More specifically, the stretched film has an elongation of 1100% to 1450% and a stiffness of 880 MPa to 950 MPa, as measured in accordance with ASTM D 882.

Further, the stretched film has a maximum stretching ratio of 6X10 or more in the machine direction (MD) and transverse direction (TD), and a stiffness of 800 MPa to 1000 MPa, or 830 MPa to 950 MPa in the MD direction, as measured after stretching at the maximum stretching ratio. More specifically, the stretched film may have a maximum stretching ratio of 6 or more, or 6 to 8 in the MD direction, a maximum stretching ratio of 10 or more, or 10 to 12 in the TD direction, and more specifically, a maximum stretching ratio of 6 in the MD direction, and a maximum stretching ratio of 10 in the TD direction. Further, the maximum stretching ratio, specifically, the maximum stretching ratio in the MD direction is 6 or more, or 6 to 8, and the maximum stretching ratio in the TD direction is 10 or more, or 10 to 12, and the stiffness in the MD direction is 800 MPa to 1000 MPa or 830 MPa to 950 MPa, as measured after stretching.

Hereinafter, preferred exemplary embodiments will be provided for better understanding of the present invention. However, the following exemplary embodiments are provided only for understanding the present invention more easily, but the content of the present invention is not limited thereby.

### <Preparation of Catalyst>

### Preparation Example 1

34 g of anhydrous magnesium chloride (99% by weight or more, a moisture content of less than 1%) was put in a 2 liter Buchi reactor dried with nitrogen, and 600 ml of purified hexane with a moisture content of less than 0.5 ppm was also put in the reactor. To the resulting reaction product, 175 ml of anhydrous 2-ethyl-1-hexanol was added under stirring, and then stirred at a temperature of 130°C for about 2 hours to prepare a homogeneous solution of magnesium carrier.

While stirring the homogeneous solution at a temperature of 35°C and a speed of 200 rpm, 200 ml of TiCl₄ was slowly added over 1 hour and stirred for another hour. In this process, a solid material was produced. After the solid material was precipitated, the liquid portion was removed, and the separated solid material was washed several times with hexane until the titanium concentration in the washing solution was 0.5 mmol or less. To the washed solid material, purified hexane was added to a total volume of 1 liter. The titanium concentration in the resulting hexane slurry was 30 mM. To the hexane slurry, 7 ml of cyclohexyl chloride was added at a temperature of 40°C, and stirred for 1 hour to obtain a Ziegler-Natta catalyst supported on magnesium chloride.

The prepared catalyst included TiCl₄ as a main catalyst, and also included cyclohexyl chloride at a molar ratio of 0.13, based on 1 mol of the main catalyst. In addition, a molar ratio of Mg:Ti in the catalyst was 4: 1, and the average particle size of catalyst particles was 8 µm.

### Preparation Example 2

A Ziegler-Natta catalyst supported on magnesium chloride was prepared in the same manner as in Preparation Example 1, except that anhydrous magnesium chloride was changed to magnesium ethylate.

The prepared catalyst included TiCl₄ as a main catalyst, and also included cyclohexyl chloride at a molar ratio of 0.13, based on 1 mol of the main catalyst. In addition, a molar ratio of Mg:Ti in the catalyst was 5:1, and the average particle size of catalyst particles was 10 µm.

### Preparation Example 3

A Ziegler-Natta catalyst supported on magnesium chloride was prepared in the same manner as in Preparation Example 1, except that anhydrous 2-ethyl-1-hexanol was changed to ethanol.

The prepared catalyst included TiCl₄ as a main catalyst, and also included cyclohexyl chloride at a molar ratio of 0.13, based on 1 mol of the main catalyst. In addition, a molar ratio of Mg:Ti in the catalyst was 4.5:1, and the average particle size of catalyst particles was 13 µm.

### <Preparation of Polyethylene Resin Composition>

### Example 1

In a bimodal slurry process using two 100 L continuous stirred tank reactors (CSTR), a high density polyethylene resin composition was prepared by a method of preparing polyethylene by polymerization reaction in the presence of a Ziegler-Natta catalyst system.

In detail, in a first reactor, a first polyethylene was prepared by performing a primary polymerization reaction in the presence of ethylene, the Ziegler-Natta catalyst prepared in Preparation Example 1, an aqueous TEAL solution (30 mmol/hr) as a cocatalyst, hydrogen, and a first comonomer under conditions shown in Table 1 below.

Next, in a second reactor, a second polymerization reaction was performed under conditions shown in Table 1 below. In this regard, the first polyethylene polymerized in the first reactor was fed to the second reactor connected in series to undergo secondary polymerization.

Based on the total weight of the polymerization product obtained as a result of the secondary polymerization, 250 ppm of BASF's Irganox 1010 as a primary antioxidant, 500 ppm of BASF's Irgafos 168 as a secondary antioxidant, 5000 ppm of calcium stearate (Ca-St) as a neutralizing were fed and mixed, and then extruded at an extrusion temperature of 190°C using a twin screw extruder (TEK 30 MHS, manufactured by SMPLATECH CO., diameter of 32 pi, LID = 40) to prepare a pellet-type polyethylene resin composition.

### Examples 2 and 3 and Comparative Examples 1 to 8

Each polyethylene resin composition was prepared in the same manner as in Example 1, except that conditions were changed as in Tables 1 or 2, below.

Meanwhile, in Comparative Example 3, a polyethylene resin composition was prepared by a monomodal slurry process of using a single CSTR.

**[Table 1]**

| | | Example | | |
|---|---|---|---|---|
| | | 1 | 2 | 3 |
| First reactor | Input amount of ethylene [kg/hr] | 5.5 | 5.5 | 5.5 |
| | Type of catalyst | Preparation Example 1 | Preparation Example 2 | Preparation Example 3 |
| | Input amount of catalyst [ml/hr] | 50 | 50 | 50 |
| | Type of first comonomer | 1-butene | 1-butene | 1-butene |
| | Input amount of first comonomer [ml/min] | 5.5 | 6 | 6.5 |
| | Hydrogen [g/hr] | 8 | 7 | 8 |
| | Temperature (T1) [°C] | 80 | 80 | 80 |
| | Pressure (P1) [kgf/cm²] | 6.7 | 6.3 | 7.1 |
| Second reactor | Ethylene [kg/hr] | 4.5 | 4.5 | 4.5 |
| | Input amount of catalyst [ml/hr] | 0 | 0 | 0 |
| | Type of second comonomer | - | - | 1-octene |
| | Input amount of second comonomer [ml/min] | 0 | 0 | 1.5 |
| | Hydrogen [g/hr] | 0 | 0 | 0 |
| | Temperature (T2) [°C] | 75 | 75 | 75 |
| | Pressure (P2) [kgf/cm²] | 3.1 | 2.6 | 2.2 |
| Weight ratio of ethylene: comonomer | | 1:0.020 | 1:0.022 | 1:0.031 |
| Input amount of hydrogen [wt%] | | 0.08 | 0.07 | 0.08 |

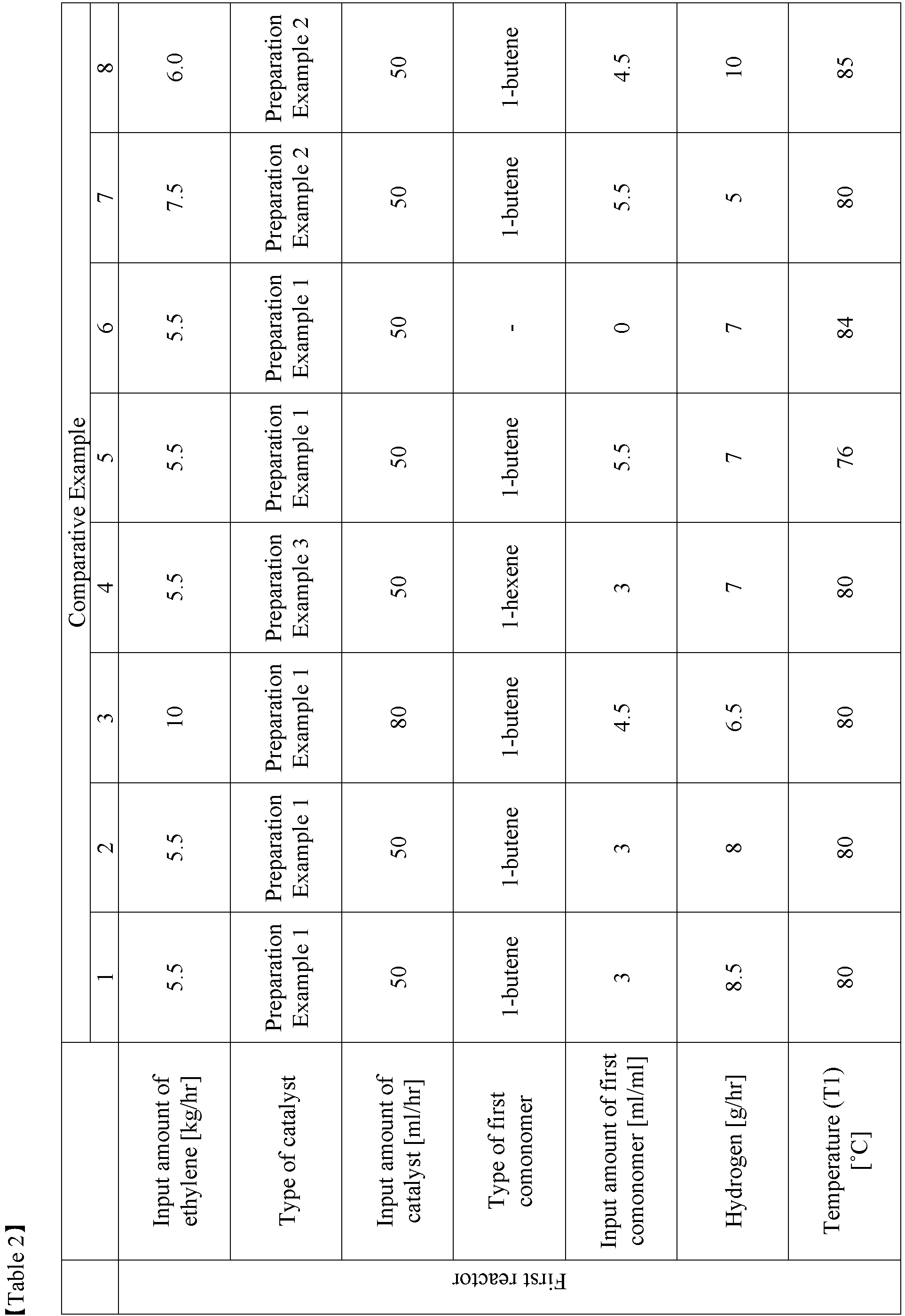

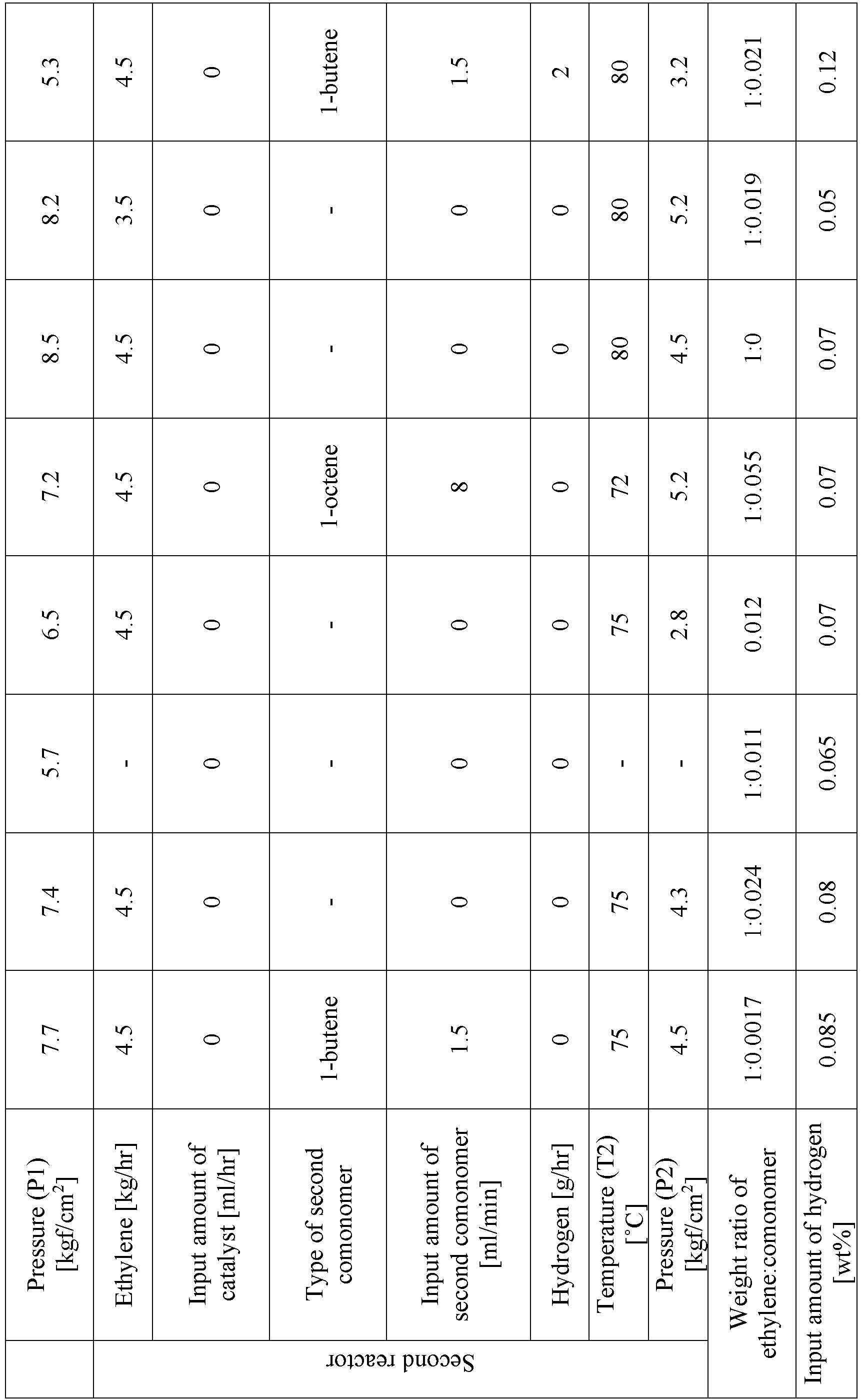

In Table 1, the weight ratio of ethylene:comonomer is a weight ratio of the total amount of ethylene introduced into the first and second reactors and the total amount of comonomers including the first and second comonomers.

Further, the input amount of hydrogen (wt%) is expressed as a percentage of the total weight of hydrogen input, based on the total weight of ethylene introduced into the first and second reactors.

### Experimental Example 1

The physical properties of the polyethylene resin compositions prepared in Examples and Comparative Examples were measured as follows, and the results are shown in Table 3 below.
(1) Density: measured in accordance with the ASTM D1505 standard.
(2) Melt Index (MI_{2.16}): measured in accordance with the ASTM D1238 (condition E, 190 °C, under a load of 2.16 kg) standard.
(3) MFRR (MI_{21.6}/MI_{2.16}): a ratio obtained by dividing MI_{21.6} (ASTM D1238, 190°C, under a load of 21.6 kg) by MI_{2.16} (ASTM D1238, 190°C, under a load of 2.16 kg).
(4) Crystal content according to melting temperature

The polyethylene was initially heated to 160°C using a differential scanning calorimeter (device name: DSC8000, manufacturer: PerkinElmer), and then held for 30 minutes to remove all the thermal history of the sample before measurement.

The temperature was lowered from 160°C to 122°C and held for 20 minutes, and then lowered to 30°C and held for 1 minute, and then the temperature was increased again. Next, the polyethylene was heated to a temperature (117°C), which was 5°C lower than the initial heating temperature of 122°C, held for 20 minutes, and the temperature was lowered to 30°C. After holding for 1 minute, the temperature was increased again. In this manner, the n+1^{th} heating temperature was held at a temperature of 5°C lower than the n^{th} heating temperature, the holding time and cooling temperature were the same, and the heating temperature was gradually lowered to 52°C. At this time, the heating and cooling rates were adjusted to 20°C/min, respectively. Lastly, the temperature was increased from 30°C to 160°C at a heating rate of 10°C/min and an SSA thermogram was measured by observing a change of heat quantity.

The peak area was quantified using Tm and heat quantity (area Sᵢ) of each melting peak from the measured SSA thermogram. That is, as in the following equations (1) to (3), the content ratio of each area was quantified by a ratio of each of the three areas, S₁ (Tm of 100°C or lower), S₂ (Tm of higher than 100°C and lower than 120°C), and S₃ (Tm of 120°C or higher) to the area of the melting peaks (S₁+S₂+S₃) in the entire SSA thermogram.
(1) low-crystalline polymer content (W_{LC}) of Tm of 100°C or lower = S₁ / (S₁+S₂+S₃)
(2) medium-crystalline polymer content (W_{MC}) of Tm of higher than 100°C and lower than 120°C = S₂ / (S₁+S₂+S₃)
(3) high-crystalline polymer content (W_{HC}) of Tm of 120°C or higher = S₃ / (S₁+S₂+S₃)

Further, from the values calculated above, a ratio (W_{LC}/W_{HC}) of the low-crystalline polymer content (W_{LC}) in the low-temperature range of Tm of 100°C or lower to the high-crystalline polymer content (W_{HC}) in the high-temperature range of Tm is 120°C or higher was obtained.

Further, the results of SSA analysis of the polyethylene resin composition according to Example 1 are shown in FIG. 1.

### (4) Stress relaxation time (sec)

0.5 mm-thick sheets were manufactured using each of the polyethylene resin compositions of Examples and Comparative Examples using a hot press (190°C), and then cut in accordance with the ASTM D 882 standard to manufacture PE specimens.

Each PE specimen thus manufactured was fixed horizontally using a dynamic mechanical analysis (DMA) device, and a stress test was performed by pulling the lower part of the PE sheet with 1% stress for a stress application time of 25 minutes at 90°C under a tensile mode. From the results, a graph having time (t) on the x-axis and stress on the y-axis was derived. From the above graph, the time taken for the stress reduction rate to reach 0.1% compared to the initial stress was measured, and expressed as a stress relaxation time.

**[Table 3]**

| | Density (g/cm³) | MI_{2.16} (g/ 10min) | MFRR | W_{LC} | W_{MC} | W_{HC} | W_{LC}/ W_{HC} | Stress relaxation time (sec) |
|---|---|---|---|---|---|---|---|---|
| Example 1 | 0.950 | 1.24 | 4.52 | 0.083 | 0.206 | 0.711 | 0.117 | 55 |
| Example 2 | 0.949 | 0.97 | 4.13 | 0.092 | 0.04 | 0.868 | 0.106 | 58 |
| Example 3 | 0.949 | 1.12 | 4.00 | 0.091 | 0.22 | 0.689 | 0.132 | 42 |
| Comparative Example 1 | 0.954 | 0.72 | 4.16 | 0.074 | 0.147 | 0.779 | 0.095 | 68 |
| Comparative Example 2 | 0.954 | 1.19 | 4.11 | 0.072 | 0.201 | 0.727 | 0.099 | 66 |
| Comparative Example 3 | 0.951 | 0.83 | 3.83 | 0.062 | 0.026 | 0.912 | 0.068 | 52 |
| Comparative Example 4 | 0.953 | 0.90 | 3.89 | 0.056 | 0.158 | 0.786 | 0.071 | 62 |
| Comparative Example 5 | 0.925 | 1.66 | 3.02 | 0.246 | 0.257 | 0.497 | 0.495 | 24 |
| Comparative Example 6 | 0.969 | 1.12 | 3.35 | 0.025 | 0.088 | 0.887 | 0.028 | 82 |
| Comparative Example 7 | 0.960 | 0.03 | 3.2 | 0.003 | 0.066 | 0.931 | 0.003 | 97 |
| Comparative Example 8 | 0.952 | 17 | 5.9 | 0.127 | 0.35 | 0.523 | 0.243 | 45 |

As the low/high crystalline ratio (W_{LC}/W_{HC}) is higher, it is more favorable to the stretching because the crystals are in a molten state during the stretching process. In addition, as the faster relaxation occurs at the beginning, the polymer mobility is higher during stretching, which is advantageous for crystal structure rearrangement and stretching.

### Experimental Example 2

A biaxially stretched film was manufactured using each of the polyethylene resin compositions prepared in Examples and Comparative Examples, and the following physical properties were evaluated. The results are shown in Table 4.

### (1) Production of biaxially stretched film

- A cast sheet of the polyethylene resin composition was produced at a thickness of 0.72 mm using Bruckner's lab extruder line (LID ratio: 42, Screw diameter: 25 mm, Melt/T-Die temperature: 250°C).
- Biaxial stretching was performed to produce a polyethylene resin composition sheet with a width x a height of 90 mm x 90 mm using KARO 5.0 machine.
- Stretching (MD → TD) was sequentially performed after preheating for 100 seconds at 120 °C, respectively.
- When stretching, the speed was 300%/s.
- Final film thickness (based on stretching ratio of 5X8 times): 20 µm

### (2) Elongation (%) and Stiffness (MPa)

Elongation (%) and stiffness (MPa) were measured in accordance with ASTM D 882.

In detail, each of the polyethylene resin compositions of Examples and Comparative Examples was manufactured into a 2 mm-thick sheet by hot pressing (190°C), and then cut in accordance with the ASTM D 882 standard to manufacture each PE specimen.

Each PE specimen thus manufactured was fixed horizontally using a Zwick tensile tester (UTM), and a tensile test was performed by pulling the upper part of the PE sheet at room temperature (23±5°C).

The 1% secant modulus when pulled at a speed of 5 mm/min was expressed as stiffness, and the break-up strain (%) when pulled at a speed of 500 mm/min was expressed as elongation.

### (3) Maximum stretching ratio

When the casting film manufactured in (1) was subjected to biaxial stretching using Bruckner's Lab Stretcher KARO 5.0 film biaxial stretching machine, the maximum stretching ratio in the MD/TD directions at which the film was stretched without breaking or defecting was measured.

### <Measurement conditions>

Sample size : 90 mm x 90 mm
Stretch profile : sequential
Stretch rate : MD direction 5~6 times, TD direction 4~9 times
Stretch speed : 100-400%/s
Stretch temp : 110-130°C
Preheat: 100 s

### (4) MD Stiffness (MPa)

For the films of Examples and Comparative Examples which were stretched at the maximum stretching ratio in the test of measuring the maximum stretching ratio in (3), a Zwick tensile tester (UTM) was used in the same manner as the tensile test in (1) to perform a tensile modulus test at room temperature (23±5°C) in the MD direction and the 1% secant modulus was measured when pulled at a speed of 5 mm/min.

**[Table 4]**

| | Elongation (%) | Stiffness (MPa) | Maximum stretching ratio (MDxTD) | MD Stiffness (MPa) |
|---|---|---|---|---|
| Example 1 | 1216.7 | 887.5 | 6x10 | 832 |
| Example 2 | 1133.3 | 947.6 | 6x10 | 902 |
| Example 3 | 1412.8 | 926.9 | 6x10 | 875 |
| Comparative Example 1 | 762.4 | 912.1 | 4x8 | 861 |
| Comparative Example 2 | 925.4 | 792.3 | 4x8 | 698 |
| Comparative Example 3 | 382.8 | 765.4 | 5x9 | 726 |
| Comparative Example 4 | 901.5 | 967.1 | 5x9 | 921 |
| Comparative Example 5 | 1080.3 | 206.8 | 7x10 | 162 |
| Comparative Example 6 | 56.5 | 1468 | 5x9 | 1392 |
| Comparative Example 7 | 127.6 | 1275 | 4x7 | 1103 |
| Comparative Example 8 | 1534.3 | 563.8 | 6x10 | 421 |

As a result of the experiment, the resin compositions of Examples 1 to 3 according to the present invention exhibited excellent stretchability and physical properties when manufacturing stretched films, as compared to the resin compositions of Comparative Examples. Specifically, as the relaxation time decreased, the orientation and rearrangement of crystals during stretching occurred quickly, and thus the stretchability in the TD direction was improved when the film was biaxially stretched. Due to the increased crystal content in the low-temperature region, the resin compositions also exhibited excellent characteristics in terms of the maximum stretching ratio. In contrast, in the case of Comparative Example 8 having the same maximum stretching ratio, the stiffness of the resin and film greatly deteriorated due to the decreased crystal content in the high temperature region.

## Claims

1. A polyethylene resin composition comprising an ethylene/C4 to C10 alpha olefin copolymer and satisfying the following requirements of (a1) to (a3):
(a1) when a relative content of a peak area according to a melting temperature (Tm) (°C) is measured using SSA (Successive Self-nucleation and Annealing) analysis, a ratio (W_{LC}/W_{HC}) of a low-crystalline polymer content (W_{LC}) in a low temperature range of Tm of 100°C or lower to a high-crystalline polymer content (W_{HC}) in a high temperature range of Tm of 120°C or higher: 0.1 or more,
(a2) a density measured in accordance with ASTM D1505 standard: 0.940 g/cm³ or more, and
(a3) a melt index (MI_{2.16}) measured at a temperature of 190°C under a load of 2.16 kg in accordance with ASTM D1238 standard: 0.1 g/lOmin to 10 g/lOmin.

2. The polyethylene resin composition of claim 1, wherein the W_{LC}/W_{HC} of the polyethylene resin composition is 0.1 or more, and 1.5 or less.

3. The polyethylene resin composition of claim 1, wherein when the relative content of the peak area according to the melting temperature (Tm) is measured using the SSA (Successive Self-nucleation and Annealing) analysis, the high-crystalline polymer content (W_{HC}) which is defined as a ratio of peak area in the high temperature range of Tm of 120°C or higher, relative to an entire peak area, is 0.5 to 0.9.

4. The polyethylene resin composition of claim 1, wherein when the relative content of the peak area according to the melting temperature (Tm) is measured using the SSA (Successive Self-nucleation and Annealing) analysis, the low-crystalline polymer content (W_{LC}) which is defined as a ratio of peak area in the low temperature range of Tm of 100°C or lower, relative to an entire peak area, is 0.08 to 0.1.

5. The polyethylene resin composition of claim 1, wherein when the relative content of the peak area according to the melting temperature (Tm) is measured using the SSA (Successive Self-nucleation and Annealing) analysis, a medium-crystalline polymer content (W_{MC}) which is defined as a ratio of peak area in a temperature range of Tm of higher than 100°C and lower than 120°C, relative to an entire peak area, is 0.01 to 0.25.

6. The polyethylene resin composition of claim 1, wherein the SSA analysis is performed by heating the polyethylene resin composition to a first heating temperature of 140°C to 180°C using a differential scanning calorimeter, holding for 15 minutes to 30 minutes, and then cooling at 28°C to 32°C, and repeating heating-holding-cooling until a final heating temperature reaches 50°C to 54°C while lowering heating temperature stepwise wherein the n+1^{th} heating temperature is set to 3 °C to 7°C lower than the n^{th} heating temperature, and lastly, cooling from 50°C to 30°C, and then heating to 180°C.

7. The polyethylene resin composition of claim 1, wherein a density of the polyethylene resin composition is 0.945 g/cm³ or more, and 0.950 g/cm³ or less.

8. The polyethylene resin composition of claim 1, wherein a melt index (MI_{2.16}) of the polyethylene resin composition is 0.95 g/lOmin or more, and 1.5 g/lOmin or less, as measured at a temperature of 190°C under a load of 2.16 kg in accordance with ASTM D1238 standard.

9. The polyethylene resin composition of claim 1, wherein a MFRR (MI₅/MI_{2.16}) of the polyethylene resin composition is 3 or more, and 10 or less, which is a ratio obtained by dividing a melt index (MI₅) measured at a temperature of 190°C under a load of 5 kg by a melt index (MI_{2.16}) measured at a temperature of 190°C under a load of 2.16 kg in accordance with ASTM D1238 standard.

10. The polyethylene resin composition of claim 1, wherein when the polyethylene resin composition is subjected to a stress test under conditions of 90°C, 1% stress, and a stress application time of 25 minutes, the stress relaxation time required for a stress reduction rate to reach 0.1% is 60 seconds or less.

11. The polyethylene resin composition of claim 1, wherein the ethylene/C4 to C10 alpha olefin copolymer is an ethylene/1-butene copolymer or an ethylene/1-butene/1-octene copolymer.

12. A stretched film comprising the polyethylene resin composition of claim 1.

13. The stretched film of claim 12, wherein the stretched film has an elongation of 1000% to 1500% and a stiffness of 850 MPa to 1000 MPa, as measured in accordance with ASTM D 882.

14. The stretched film of claim 12, wherein the stretched film has a maximum stretching ratio of 6X10 or more in the MD and TD directions, and a stiffness of 800 MPa to 1000 MPa in the MD direction, as measured after stretching at the maximum stretching ratio.
